# EUROPEAN PATENT SPECIFICATION

(11) **EP 4 173 590 B1**
(45) Date of publication and mention of the grant of the patent: **21.05.2025**
(21) Application number: 21306486.8
(22) Date of filing: 26.10.2021
(51) Int. Cl.: A61B 90/00, A61B 34/20, A61B 17/00

(54) **METHOD FOR REGISTERING A 3D MEDICAL IMAGE WITH A REGISTRATION PHANTOM**
VERFAHREN ZUR REGISTRIERUNG EINES MEDIZINISCHEN 3D-BILDES MIT EINEM REGISTRIERUNGSPHANTOM
PROCÉDÉ D'ENREGISTREMENT D'UNE IMAGE MÉDICALE EN 3D À L'AIDE D'UN FANTÔME D'ENREGISTREMENT

(43) Date of publication of application: 03.05.2023
(73) Proprietor: Ecential Robotics, 38610 Gieres (FR)
(72) Inventor: LAVALLEE, Stéphane, 38610 Gieres (FR); VIDAL, Clément, 38610 Gieres (FR); PIERRE, Arnaud, 38610 Gieres (FR)
(74) Representative: Regimbeau

(56) References cited:
- EP-A1- 3 821 844
- US-A1- 2011 313 285
- US-A1- 2015 085 981
- US-A1- 2017 095 296
- US-A1- 2020 237 326

## Description

### TECHNICAL FIELD

The present disclosure relates to a method for registering a 3D medical image with a registration phantom, and an intraoperative surgical system configured to implement such a method.

### TECHNICAL BACKGROUND

X-Ray imaging systems are frequently used during medical surgical procedures to provide physicians with image-based information about the anatomical situation and/or the position and orientation of surgical instruments.

Such X-ray imaging systems typically provide two-dimensional (2D) projection images with different structures superimposed along the path of the X-rays.

A typical example of an X-ray imaging system for use in an intra-operative setting is the so-called C-arm used in a mobile or stationary manner and essentially consisting of a base frame on which a C-shaped arm is attached with several intermediate joints allowing moving the C-shaped arm in space along several degrees of freedom.

One end of the C-shaped arm carries an X-ray source and the other end an image detector.

Due to the limited information provided by these 2D images, three-dimensional (3D) imaging techniques have become indispensable over the past decades.

While computer tomography (CT) systems are a well-established class of stationary X-ray imaging systems used for reconstruction of a 3D image in a radiology department, these systems are in general not usable inside the operating room.

Recent years have seen an increasing interest in tomographic reconstruction techniques, also known as cone-beam reconstruction techniques (CBCT), using two-dimensional image detectors.

Special efforts have been made to enable the aforementioned C-arms to provide three-dimensional information by automatically acquiring a set of 2D images of a region of interest and subsequently reconstructing a 3D image based on said cone-beam reconstruction techniques.

Surgical navigation is the application of real-time navigation on intraoperatively acquired fluoroscopic images to achieve the above-mentioned goals.

In view of carrying out said navigation, the tools used during the surgical intervention are equipped with a tracker, e.g. an optical, electromagnetic, ultrasonic, or inertial tracker which is tracked in real time by at least one localization system. Another tracker is mounted onto the patient and is also tracked in real time by the localization system. In this way, the position and orientation of the tools relative to the patient is known in real time.

In view of navigating surgical tools with respect to images acquired by the X-ray imaging device, it is necessary to know the position of said images (or the position the X-ray image detector during acquisition of said images) with respect to the tracker fixed on the patient. To that end, an instrument may be used such as described in [1].

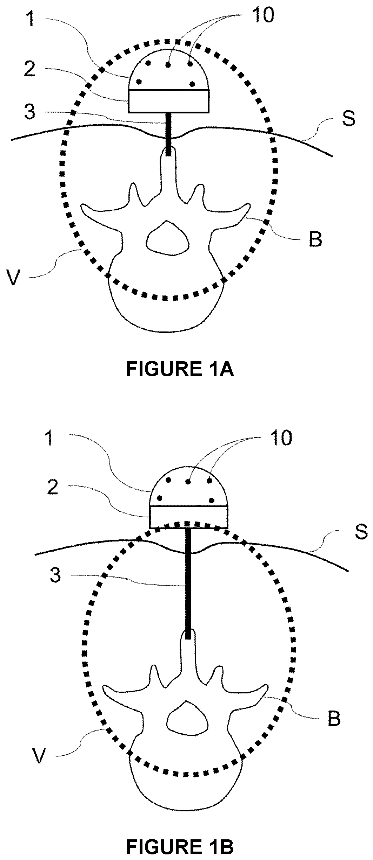

As shown in FIG. 1A, the instrument comprises a registration phantom 1 comprising a plurality of radiopaque fiducials 10 arranged according a known geometry, such that the position of each radiopaque fiducial is known in a coordinate system of the registration phantom. The instrument also comprises a base 2 supporting the registration phantom 1. Preferably, the registration phantom 1 can be removed from the base 2, and a tracker (not shown) can instead be fixed to the base 2 to allow tracking the patient during surgery. The base 2 is rigidly fixed to a patient's bone by at least one percutaneous pin 3, the base 2 and the registration phantom 1 being located outside the patient's body, above the patient's skin S. The dotted line schematically represents the volume V that is reconstructed from 2D X-ray images of the patient. Since said volume V includes not only the region of interest, which is a part of the bone, but also the registration phantom 1, the radiopaque fiducials 10 that are visible in the 3D image allow determining the position of the volume V in the coordinate system of the registration phantom and thus register the 3D image with the registration phantom. Since the tracker is fixed to the same base as the registration phantom, it is thus possible to navigate a surgical instrument within the 3D image.

But sometimes, especially if the patient is obese, the distance between the registration phantom and the region of interest to image is such that said phantom lies outside the reconstructed volume. In that case, traditional methods prove ineffective to register the imaging space with the navigation space.

This situation is illustrated in FIG. 1B. Due to the larger thickness of tissues between the bone B and the skin S, the base 2 and registration phantom 1 are located farther from the bone B. Thus, for a same volume V reconstructed from the 2D X-ray images, the registration phantom 1 is located outside the volume V. Since the radiopaque fiducials 10 are not inside the volume V, it is not possible to determine the position of the volume V in the coordinate system of the registration phantom and thus register the 3D image with the registration phantom.

Document US 2015/085981 teaches a method for registering a 3D image with a 2D image which does not require any marker. The method uses a registration algorithm that solves the transformation of a 3D image (which may be acquired preoperatively or intraoperatively) such that a 2D projection computed from the 3D image (in particular, a digitally reconstructed radiograph, DRR) yields maximum similarity to an intraoperative 2D X-ray image.

Document US 2017/095296 relates to a method for imaging a catheter or a needle within a patient's vascular structure.

Document US 2020/237326 relates to a method for determining an orientation of a needle in a 3D medical image.

### SUMMARY OF THE DISCLOSURE

It is thus desirable to define a method for registering a 3D medical image obtained by an X-ray imaging system with a registration phantom comprising radiopaque fiducials even if the registration phantom is located relative to the patient such that the radiopaque fiducials are not visible in the 3D medical image.

An object of the present disclosure is thus a method for registering a 3D medical image obtained by an X-ray imaging system with a registration phantom, wherein the registration phantom is positioned onto the patient and comprises a set of radiopaque fiducials having a known position in a coordinate system of said registration phantom, said set of radiopaque fiducials not being detectable in the 3D image, the method comprising the steps of:
- obtaining at least two 2D X-ray images acquired by the X-ray imaging system wherein at least one radiopaque fiducial of the registration phantom is detectable in each 2D image;
- for each of said at least two 2D images, registering the 2D X-ray image with the registration phantom by detecting and determining the position of the at least one radiopaque fiducial in the respective 2D image;
- registering each of said at least two 2D X-ray images with the 3D image using an image-to-image registration technique;
- registering the registration phantom with the 3D medical image using said at least two 2D X-ray images.

In preferred embodiments, an anatomical region of interest of the patient is detectable in each of the at least two 2D images and in the 3D image, and the image-to-image registration technique computes an optimization between a projection of said region of interest in the 3D image onto the image detector and said region of interest in each of the 2D X-ray images.

In some embodiments, the 3D medical image is reconstructed from a set of 2D images acquired by the X-ray imaging system and the at least two 2D images are selected from said set of 2D images.

In other embodiments, the 3D medical image is reconstructed from a set of 2D images acquired by the X-ray imaging system and the 2D images are acquired with the X-ray imaging system after reconstructing the 3D medical image.

In other embodiments, wherein the 3D medical image is reconstructed from a set of 2D images acquired by the X-ray imaging system and the 2D images are acquired with the X-ray imaging system before reconstructing the 3D medical image.

The image-to-image registration technique can typically comprise at least one of the following techniques: cross-correlation techniques, mutual information techniques, gradient correlation techniques.

Another object is an intraoperative surgical system comprising:
- a registration phantom comprising a set of radiopaque fiducials having a known position in a coordinate system of said registration phantom, said registration phantom being adapted to be positioned onto a patient,
- an X-ray imaging system configured to acquire a set of 2D X-ray images and to reconstruct a 3D medical image from said set of 2D X-ray images,
- a computer system configured to:
   (i) obtain at least two 2D X-ray images acquired by the X-ray imaging system wherein at least one radiopaque fiducial (10) of the registration phantom (1) is detectable in each 2D image;
   (ii) for each of said at least two 2D images, register the 2D X-ray image with the registration phantom by detecting and determining the position of the at least one radiopaque fiducial in the respective 2D image;
   (iii) register each of said at least two 2D X-ray images with the 3D image using an image-to-image registration technique;
   (iv) register the registration phantom with the 3D medical image using said at least two 2D X-ray images.

In preferred embodiments, the X-ray imaging system is a motorized C-arm.

In some embodiments, the system further comprises a radiotransparent base configured to be attached to the patient's anatomy, said base comprising a fixation system for removably attaching the registration phantom to the base.

The system may further comprise a localization system and at least one tracker adapted to be tracked by the localization system, the base comprising a fixation system for removably attaching the tracker to the base.

### BRIEF DESCRIPTION OF THE FIGURES

Further features and advantages of the method and the system will be apparent from the following detailed description, based on the appended drawings, in which:
- FIG. 1A is a schematic view of a registration phantom close enough to the region of interest to allow registering a 3D image of the region of interest with the coordinate system of the registration phantom by detecting the radiopaque fiducials of the registration phantom in the 3D image;
- FIG. 1B is a schematic view of a registration phantom too far from the region of interest to allow registering a 3D image of the region of interest with the coordinate system of the registration phantom by detecting radiopaque fiducials of the registration phantom in the 3D image;
- FIG. 2 represents a surgical scene comprising an intraoperative X-ray imaging system and a patient lying on an operating table;
- FIG. 3 schematically illustrates the acquisition of two 2D X-ray images in which the radiopaque fiducials of the registration phantom are visible;
- FIG. 4 schematically illustrates the registration of a 2D X-ray image with the registration phantom;
- FIG. 5 schematically illustrates the registration of a 2D X-ray image with the 3D image.

For sake of clarity, the figures are not necessarily drawn to scale.

The reference signs identical from one figure to another one designate the same elements or elements having the same function. Thus, these elements may not be described in detail again.

### DETAILED DESCRIPTION OF EMBODIMENTS

The registration method is carried out thanks to a computer system which comprises at least one processor configured to implement algorithms, at least one data storage device, a communication module and at least one user interface.

The intraoperative surgical system also comprises an X-ray imaging system configured to reconstruct a 3D image from a set of 2D X-ray images, in particular by CBCT.

In some embodiments, the computer system may be the control unit of the X-ray imaging system.

In other embodiments, the computer system may be coupled to the control unit of the X-ray imaging system, so as to receive 2D and/or 3D images from the X-ray imaging system. The computer system may also send commands to the control unit of the X-ray imaging system to acquire additional 2D X-ray images, in particular when such additional images are necessary to perform the registration of the 3D image with the registration phantom, as will be described below.

In other embodiments, the computer system may not be coupled to the control unit of the X-ray imaging system. In such case, all the images necessary for the implementation of the method are acquired independently by an X-ray imaging system and recorded on a suitable support, and the computer system receives said images from the support to perform the registration.

The intraoperative surgical system may also include a localization system adapted to track in real time trackers attached to the patient and to at least one surgical tool.

### Registration phantom

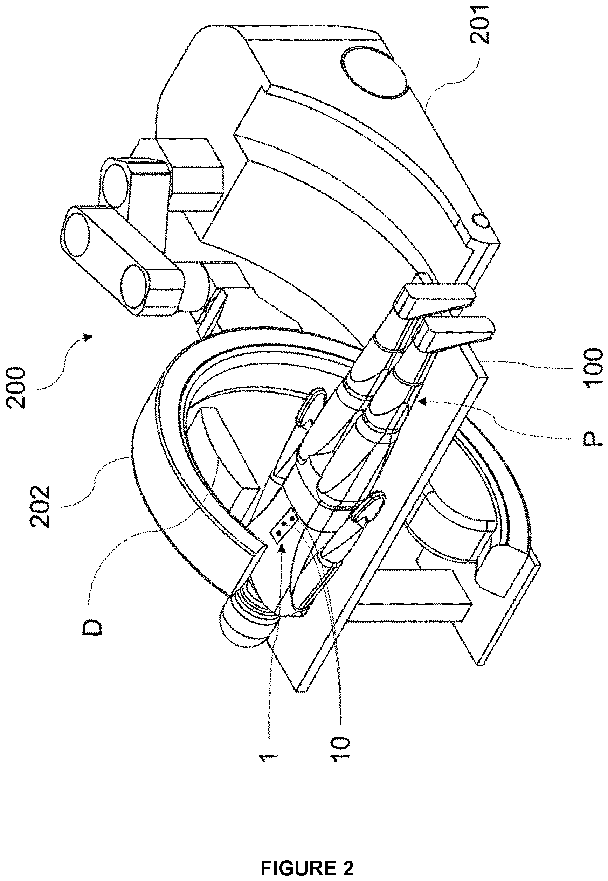

As shown in FIG. 2, at the beginning of a surgical intervention, a patient P lies on an operating table 100.

A registration phantom 1 is placed onto the patient. Preferably, the placement of the registration phantom is done without any surgical step, for example by using an adhesive to attach the registration phantom to the patient's skin, or by attaching the registration phantom to a device already implanted into a patient's bone for another purpose, such as the base intended to attach a tracker to the bone.

As shown in FIG. 1B, the registration phantom 1 comprises at least one radiopaque fiducial 10, preferably a plurality of radiopaque fiducials, in particular three or more radiopaque fiducials. Each fiducial has a known position in a 3D coordinate system of the registration phantom.

Each fiducial may be a ball-shaped or a needle-shaped piece of radiopaque material, such as steel, stainless steel, or zircon, and whose dimensions are known by any methods available to the skilled person. The fiducials are embedded in or supported by a support made of a substantially radiotransparent material. In this way, when a 2D X-ray image of the registration phantom is acquired, the fiducials are visible in the image whereas the support is substantially not visible, or at least does not hinder the detection of the fiducials in the 2D X-ray image.

The registration phantom is attached to the patient above the patient's skin, i.e. outside the patient's body.

Various ways of non-invasive or minimally-invasive fixation of the registration phantom to the patient may be used.

In some embodiments, the registration phantom may be fixed to the patient's skin by an adhesive. Although such a fixation does not provide a strictly rigid attachment to a patient's bone due to the presence of soft tissues interposed the registration phantom and the bone, it may be considered that, if the registration phantom is sufficiently close to the bone, for example just above the bone, the fixation is sufficiently rigid to provide a reasonable accuracy of the registration.

In other embodiments, the registration phantom may be fixed to a patient's bone by at least one percutaneous pin.

Preferably, the registration phantom may be attached to a base 2 of a modular device that is provided for attachment of a tracker to the patient. In such a way, the fixation of the registration phantom does not require any surgical step, but simply benefits from the base that is attached to the patient for navigation of a surgical tool.

Said modular device advantageously comprises:
- the registration phantom 1;
- the base 2, which is made of a substantially radio-transparent material, said base being configured to be rigidly secured to a patient, for example by percutaneous pins;
- a tracker (not shown) adapted to be tracker by a localization system.

The base comprises a fixation system adapted to rigidly attach the registration phantom and the tracker to the base. Preferably, the fixation system allows removing the registration phantom and/or the tracker from the base. Said fixation system is preferably designed to allow a reproducible fixation of the registration phantom and/or the tracker, i.e. to provide a same position of the registration phantom and/or the tracker relative to the base over time.

Since the registration phantom and the tracker are not used in the same steps of the surgical procedure, the base may comprise a common fixation system for interchangeably attaching the tracker and the registration phantom to the base.

### X-ray imaging system

The X-ray imaging system 200 comprises at least one X-ray source (not visible in FIG. 2) and at least one X-ray image detector D.

The X-ray imaging system is configured to produce at least one 2D X-ray image that is the result of a conical projection of a patient's anatomy, wherein the tip of the cone is approximately the central point of the X-ray source and the basis of the cone is approximately the portion of the X-ray image detector that is reached by X-ray beams that have been collimated in a given shape and orientation.

For example, the X-ray imaging system may be a conventional C-arm, or any Cone-Beam Computed Tomography (CBCT) such as the SURGIVISIO device (eCential Robotics, Gières, France), or VISON FD VARIO 3D (Ziehm), CIOS SPIN MOBILE 3D (Siemens), AIRO (Stryker), LOOP-X (Brainlab), O-ARM (Medtronic).

A CBCT-capable system has a mobile X-ray source and a mobile X-ray image detector, wherein the X-ray source and the X-ray image detector have motorized motions, moving together or independently. A CBCT system can have a C-arm shape or an O-arm shape. It can be used to acquire a set of 2D X-ray images over approximately 180° of orbital rotation that can be combined with translations and from which a 3D image can be reconstructed using tomography or tomosynthesis algorithms.

The C-shaped arm may comprise motors allowing movement horizontally, vertically and around the swivel axes, so that 2D X-ray images of the patient are produced from almost any angle. Each motor is associated to an encoder that provides at any time the relative position of the X-ray imaging system with respect to a reference position. When a 2D X-ray image is acquired, the corresponding position of the imaging system is recorded. Thus, each 2D image is recorded in a 3D coordinate system of the X-ray imaging system.

The X-ray imaging system 200 advantageously has a mobile base 201 allowing displacing the C-arm 202 in the operating room. Said mobile base may comprise control switches (not illustrated), such as a power switch, an emergency button and the like.

The acquisition of the X-ray images and the activation of the motors of the C-arm, is controlled by a control unit of the X-ray imaging system. Said control unit comprises at least one processor configured to implement algorithms, at least one data storage device, a communication module and at least one user interface.

Said control unit may be embedded in the mobile base 201 of the X-ray imaging system or in a separate cart.

### Reconstruction of the 3D image

As shown in FIG. 2, the intraoperative X-ray imaging system 200 is brought along the operating table 100 and positioned relative to a predetermined region of interest of the patient so as to be able to acquire a 3D volume of said region. The region of interest may be an anatomical structure of the patient, such as a bone or a plurality of bones, or a part of an anatomical structure.

The 3D volume is acquired intraoperatively with the X-ray imaging system through the acquisition of a set of 2D X-ray images along an acquisition path, typically evolving at least a full orbital motion.

The set of 2D images may comprise up to several hundreds of 2D images.

Tomography is then used to reconstruct a 3D volume of the region of interest from the plurality of acquired 2D X-ray images.

The reconstructed 3D volume is defined as the intersection of all the conical projections corresponding to the set of 2D images. In order to maximize the size of the 3D image of the region of interest, the acquisition of the 2D X-ray images is preferably done with an acquisition path minimizing the distance between the image detector and the patient's body. Such an acquisition path also has the advantage of moving the X-ray source away from the patient's body, which allows reducing the X-ray dose received by the patient.

However, if, as shown in FIG. 1B, the registration phantom is too far from the region of interest, it is not included in the reconstructed 3D volume. This situation may occur in particular when the patient suffers from obesity, in which case the distance between the registration phantom and the region of interest to image is so large that said phantom lies outside the reconstructed volume. Such a situation may also occur if the workflow of the surgical intervention requires that the registration phantom be placed in another position distant from the region of interest; this may happen, for example, if the registration phantom is not removable and has to be kept on the patient during the whole surgical procedure and the surgeon requests that the registration phantom be placed in a position that does not hinder the surgical gesture.

The registration of the 3D image with the registration phantom is thus carried out as follows.

### Registration of the 3D image with the registration phantom

At least two 2D X-ray images are acquired by the X-ray imaging system such that at least one radiopaque fiducial of the registration phantom is detectable in each 2D image.

In these 2D X-ray images, the region of interest is also visible. The region of interest thus forms a common feature between the 2D X-ray images and the 3D image, that will allow the registration.

In some embodiments, said at least two 2D X-ray images are selected from the set of 2D X-ray images used for the reconstruction. This allows avoiding the acquisition of additional 2D X-ray images and thus avoiding increasing the X-ray dose received by the patient. In order to maximize the accuracy of the method, said 2D X-ray images may be selected so as to maximize the number of radiopaque fiducials visible in each 2D X-ray image. Preferably, said 2D X-ray images may also be chosen to correspond to sufficiently different angles of incidence of the X-rays.

In other embodiments, said at least two 2D X-ray images are acquired with the X-ray imaging system before reconstructing the 3D medical image. In such case, the position of the X-ray source and image detector for each image acquisition is specifically chosen so as to ensure that the radiopaque fiducials of the registration phantom will be visible in each of said 2D X-ray images.

In other embodiments, said at least two 2D X-ray images are acquired with the X-ray imaging system after reconstructing the 3D medical image. In such case, the position of the X-ray source and image detector for each image acquisition is specifically chosen so as to ensure that the radiopaque fiducials of the registration phantom will be visible in each of said 2D X-ray images. Carrying out this additional acquisition after reconstructing the 3D medical image allows exposing the patient to an additional X-ray dose only if the 2D X-ray images already acquired do not allow implementing the registration method.

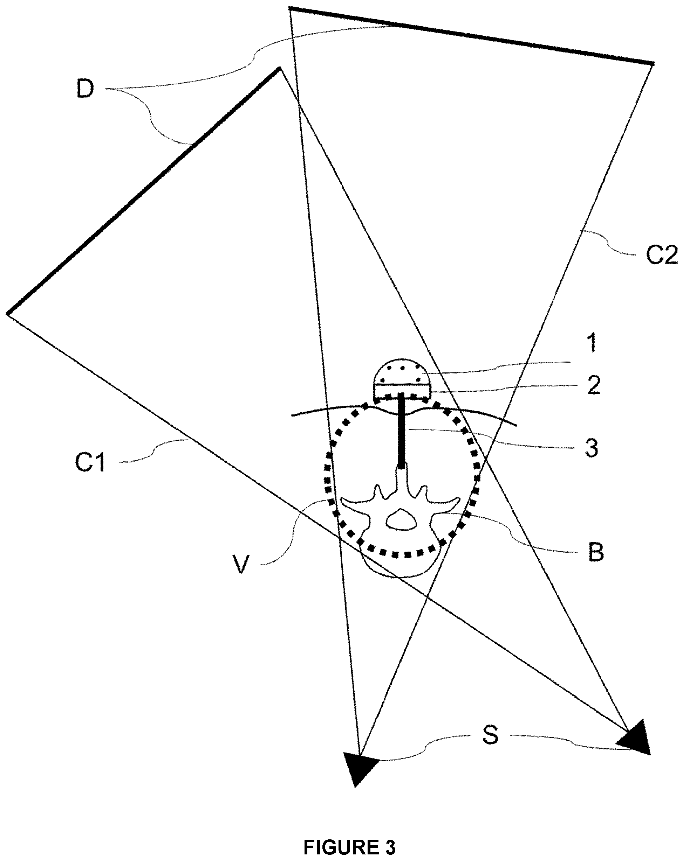

FIG. 3 schematically illustrates the acquisition of such 2D X-ray images. Each 2D X-ray image is the result of a conical projection of the patient's anatomy, wherein the tip of each cone C1, C2 is approximately the central point of the X-ray source S and the base of the cone is approximately the portion of the X-ray image detector D that is reached by X-ray beams that have been collimated in a given shape and orientation.

The method then comprises, for each of said at least two 2D images, registering the 2D X-ray image with the registration instrument by detecting and determining the position of the radiopaque fiducials in the respective 2D image.

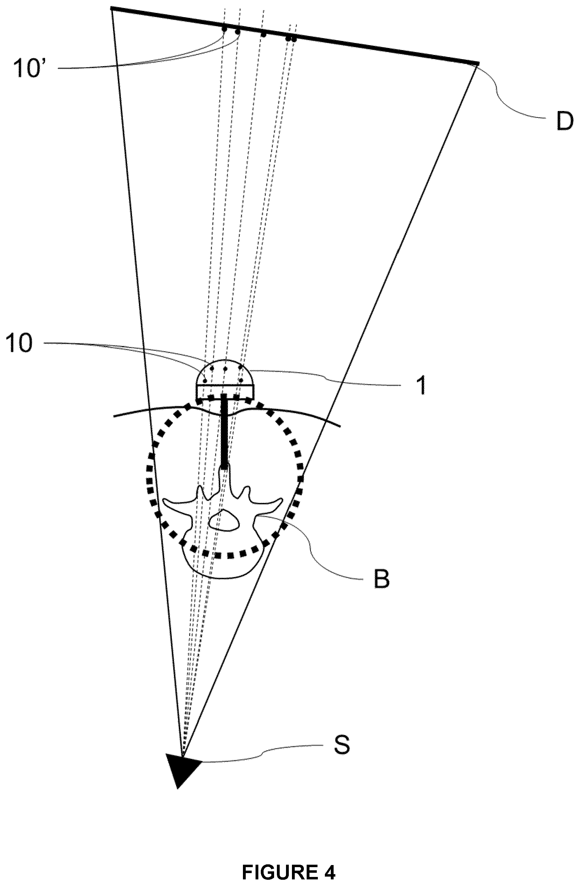

FIG. 4 schematically illustrates said registration step for one 2D X-ray image.

The dotted lines represent the direction of projection of each radiopaque fiducial 10 onto the image detector D, to form a respective spot 10' visible in the 2D X-ray image.

The detection of the radiopaque fiducials in the 2D X-ray image allows determining the position of said 2D X-ray image in the coordinate system of the registration phantom.

The method then comprises registering each 2D X-ray image with the 3D image.

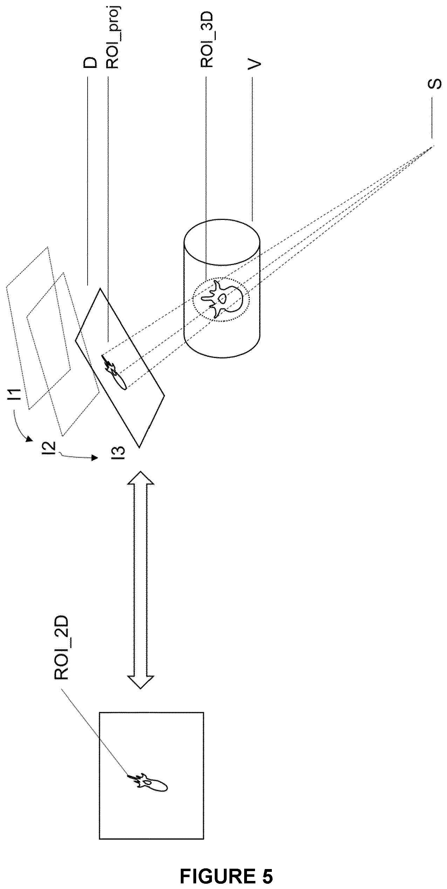

As shown in FIG. 5, said registration is based on an optimization (minimization of an error) between a projection ROI_proj of the region of interest ROI_3D of the 3D volume V onto the image detector and the region of interest ROI_2D of each 2D X-ray images. As schematically represented by iterations I1, I2, I3 of the position of the image detector, this optimization process may be iterative and stopped once the error is below a determined limited.

The method then comprises the registration of the 3D image with the registration phantom. This registration is made via the at least two 2D X-ray images, whose position is known with respect to both the registration phantom and the 3D image. It is thus possible to establish a correspondence between the 3D image and the coordinate system of the registration phantom.

### Image-to-image registration techniques

The step of registration of the at least two 2D images with the 3D volume is based on image-to-image registration techniques.

These techniques are widely known in the state of the art, and notably include:
- cross-correlation techniques
- mutual information techniques
- gradient correlation techniques.

For a review of said image-to-image registration techniques, one may refer to [2].

### REFERENCES

[1] US 2018/0280092
[2] Russakoff D.B. et al. (2003) Evaluation of Intensity-Based 2D-3D Spine Image Registration Using Clinical Gold-Standard Data. In: Gee J.C., Maintz J.B.A., Vannier M.W. (eds) Biomedical Image Registration. WBIR 2003. Lecture Notes in Computer Science, vol 2717. Springer, Berlin, Heidelberg.

## Claims

1. Method for registering a 3D medical image obtained by an X-ray imaging system with a registration phantom (1), wherein the registration phantom (1) is positioned onto the patient and comprises a set of radiopaque fiducials (10) having a known position in a coordinate system of said registration phantom (1), said set of radiopaque fiducials not being detectable in the 3D image, the method comprising the steps of:
- obtaining at least two 2D X-ray images acquired by the X-ray imaging system wherein at least one radiopaque fiducial (10) of the registration phantom (1) is detectable in each 2D image;
- for each of said at least two 2D images, registering the 2D X-ray image with the registration phantom (1) by detecting and determining the position of the at least one radiopaque fiducial (10) in the respective 2D image;
- registering each of said at least two 2D X-ray images with the 3D image using an image-to-image registration technique;
- registering the registration phantom (1) with the 3D medical image using said at least two 2D X-ray images.

2. Method according to claim 1, wherein an anatomical region of interest of the patient is detectable in each of the at least two 2D images and in the 3D image, and the image-to-image registration technique computes an optimization between a projection (ROI_proj) of said region of interest (ROI_3D) in the 3D image onto an image detector of the X-ray imaging system and said region of interest (ROI_2D) in each of the 2D X-ray images.

3. Method according to claim 1 or claim 2, wherein the 3D medical image is reconstructed from a set of 2D images acquired by the X-ray imaging system and the at least two 2D images are selected from said set of 2D images.

4. Method according to claim 1 or claim 2, wherein the 3D medical image is reconstructed from a set of 2D images acquired by the X-ray imaging system and the 2D images are acquired with the X-ray imaging system after reconstructing the 3D medical image.

5. Method according to claim 1 or claim 2, wherein the 3D medical image is reconstructed from a set of 2D images acquired by the X-ray imaging system and the 2D images are acquired with the X-ray imaging system before reconstructing the 3D medical image.

6. Method according to any one of claims 1 to 5, wherein the image-to-image registration technique comprises at least one of the following techniques: cross-correlation techniques, mutual information techniques, gradient correlation techniques.

7. Intraoperative surgical system comprising:
- a registration phantom (1) comprising a set of radiopaque fiducials (10) having a known position in a coordinate system of said registration phantom (1), said registration phantom being adapted to be positioned onto a patient,
- an X-ray imaging system configured to acquire a set of 2D X-ray images and to reconstruct a 3D medical image from said set of 2D X-ray images,
- a computer system configured to:
(i) obtain at least two 2D X-ray images acquired by the X-ray imaging system wherein at least one radiopaque fiducial (10) of the registration phantom (1) is detectable in each 2D image;
(ii) for each of said at least two 2D images, register the 2D X-ray image with the registration phantom (1) by detecting and determining the position of the at least one radiopaque fiducial (10) in the respective 2D image;
(iii) register each of said at least two 2D X-ray images with the 3D image using an image-to-image registration technique;
(iv) register the registration phantom (1) with the 3D medical image using said at least two 2D X-ray images.

8. System according to claim 7, wherein the X-ray imaging system is a motorized C-arm.

9. System according to claim 7 or claim 8, further comprising a radiotransparent base (2) configured to be attached to the patient's anatomy, said base (2) comprising a fixation system for removably attaching the registration phantom (1) to the base.

10. System according to any one of claims 7 to 9, further comprising a localization system and at least one tracker adapted to be tracked by the localization system, the base comprising a fixation system for removably attaching the tracker to the base.

## Patentansprüche

1. Verfahren zur Registrierung eines medizinischen 3D-Bildes, das von einem Röntgenbildgebungssystem erhalten wird, mit einem Registrierungsphantom (1), wobei das Registrierungsphantom (1) auf dem Patienten positioniert wird und eine Menge von strahlenundurchlässigen Fiducials (10) umfasst, die eine bekannte Position in einem Koordinatensystem des Registrierungsphantoms (1) aufweisen, wobei die Menge von strahlenundurchlässigen Fiducials nicht in dem 3D-Bild erkennbar sind, wobei das Verfahren die folgenden Schritte umfasst:
- Erhalten mindestens zweier 2D-Röntgenbilder, die von dem Röntgenbildgebungssystem erfasst werden, wobei mindestens ein strahlenundurchlässiges Fiducial (10) des Registrierungsphantoms (1) in jedem 2D-Bild erkennbar ist;
- für jedes der mindestens zwei 2D-Bilder, Registrieren des 2D-Röntgenbildes mit dem Registrierungsphantom (1) durch Erkennen und Bestimmen der Position des mindestens einen strahlenundurchlässigen Fiducials (10) in dem jeweiligen 2D-Bild;
- Registrieren jedes der mindestens zwei 2D-Röntgenbilder mit dem 3D-Bild unter Verwendung einer Bild-zu-Bild-Registrierungstechnik;
- Registrieren des Registrierungsphantoms (1) mit dem medizinischen 3D-Bild unter Verwendung der mindestens zwei 2D-Röntgenbilder.

2. Verfahren nach Anspruch 1, wobei eine anatomische Region von Interesse des Patienten in jedem der mindestens zwei 2D-Bilder und in dem 3D-Bild erkennbar ist und die Bild-zu-Bild-Registrierungstechnik eine Optimierung zwischen einer Projektion (ROI_proj) der Region von Interesse (ROI_3D) in dem 3D-Bild auf einen Bilddetektor des Röntgenbildgebungssystems und der Region von Interesse (ROI_2D) in jedem der 2D-Röntgenbilder berechnet.

3. Verfahren nach Anspruch 1 oder Anspruch 2, wobei das medizinische 3D-Bild von einer Menge von 2D-Bildern rekonstruiert wird, die von dem Röntgenbildgebungssystem erfasst werden, und die mindestens zwei 2D-Bilder von der Menge von 2D-Bildern ausgewählt werden.

4. Verfahren nach Anspruch 1 oder Anspruch 2, wobei das medizinische 3D-Bild von einer Menge von 2D-Bildern rekonstruiert wird, die von dem Röntgenbildgebungssystem erfasst werden, und die 2D-Bilder mit dem Röntgenbildgebungssystem nach dem Rekonstruieren des medizinischen 3D-Bildes erfasst werden.

5. Verfahren nach Anspruch 1 oder Anspruch 2, wobei das medizinische 3D-Bild von einer Menge von 2D-Bildern rekonstruiert wird, die von dem Röntgenbildgebungssystem erfasst werden, und die 2D-Bilder mit dem Röntgenbildgebungssystem vor dem Rekonstruieren des medizinischen 3D-Bildes erfasst werden.

6. Verfahren nach einem der Ansprüche 1 bis 5, wobei die Bild-zu-Bild-Registrierungstechnik mindestens eine der folgenden Techniken umfasst: Kreuzkorrelationstechniken, gegenseitige Informationstechniken, Gradientenkorrelationstechniken.

7. Intraoperatives chirurgisches System, umfassend:
- ein Registrierungsphantom (1), das eine Menge von strahlenundurchlässigen Fiducials (10) umfasst, die eine bekannte Position in einem Koordinatensystem des Registrierungsphantoms (1) aufweisen, wobei das Registrierungsphantom dazu geeignet ist, auf einem Patienten positioniert zu sein,
- ein Röntgenbildgebungssystem, das dazu ausgestaltet ist, eine Menge von 2D-Röntgenbildern zu erfassen und ein medizinisches 3D-Bild von der Menge von 2D-Röntgenbildern zu rekonstruieren,
- ein Computersystem, das ausgestaltet ist zum:
(i) Erhalten mindestens zweier 2D-Röntgenbider, die von dem Röntgenbildgebungssystem erfasst werden, wobei mindestens ein strahlenundurchlässiges Fiducial (10) des Registrierungsphantoms (1) in jedem 2D-Bild erkennbar ist;
(ii) für jedes der mindestens zwei 2D-Bilder, Registrieren des 2D-Bildes mit dem Registrierungsphantom (1) durch Erkennen und Bestimmen der Position des mindestens einen strahlenundurchlässigen Fiducials (10) in dem jeweiligen 2D-Bild;
(iii) Registrieren jedes der mindestens zwei 2D-Röntgenbilder mit dem 3D-Bild unter Verwendung einer Bild-zu-Bild-Registrierungstechnik;
(iv) Registrieren des Registrierungsphantoms (1) mit dem medizinischen 3D-Bild unter Verwendung der mindestens zwei 2D-Röntgenbilder.

8. System nach Anspruch 7, wobei das Röntgenbildgebungssystem ein motorisierter C-Bogen ist.

9. System nach Anspruch 7 oder Anspruch 8, ferner umfassend eine strahlendurchlässige Basis (2), die dazu ausgestaltet ist, an der Anatomie des Patienten angebracht zu sein, wobei die Basis (2) ein Fixierungssystem zum abnehmbaren Anbringen des Registrierungsphantoms (1) an der Basis umfasst.

10. System nach einem der Ansprüche 7 bis 9, ferner umfassend ein Lokalisierungssystem und mindestens einen Tracker, der dazu geeignet ist, von dem Lokalisierungssystem verfolgt zu werden, wobei die Basis ein Fixierungssystem zum abnehmbaren Anbringen des Trackers an der Basis umfasst.

## Revendications

1. Procédé de recalage d'une image médicale 3D obtenue par un système d'imagerie à rayons X avec un fantôme de recalage (1), dans lequel le fantôme de recalage (1) est positionné sur le patient et comprend un ensemble de repères radio-opaques (10) ayant une position connue dans un système de coordonnées dudit fantôme de recalage (1), ledit ensemble de repères radio-opaques n'étant pas détectable dans l'image 3D, le procédé comprenant les étapes suivantes :
- obtenir au moins deux images radiographiques 2D acquises par le système d'imagerie à rayons X, dans lesquelles au moins un repère radio-opaque (10) du fantôme de recalage (1) est détectable sur chaque image 2D ;
- pour chacune de ces au moins deux images 2D, mettre en correspondance l'image radiographique 2D avec le fantôme de recalage (1) en détectant et en déterminant la position d'au moins un repère radio-opaque (10) dans l'image 2D respective ;
- mettre en correspondance chacune de ces au moins deux images radiographiques 2D avec l'image 3D à l'aide d'une technique de recalage d'image à image ;
- mettre en correspondance le fantôme de recalage (1) avec l'image médicale 3D à l'aide d'au moins deux images radiographiques 2D.

2. Procédé selon la revendication 1, dans lequel une région anatomique d'intérêt du patient est détectable dans chacune des au moins deux images 2D et dans l'image 3D, et la technique de recalage d'image à image calcule une optimisation entre une projection (ROI_proj) de ladite région d'intérêt (ROI_3D) dans l'image 3D sur un détecteur d'image du système d'imagerie à rayons X et ladite région d'intérêt (ROI_2D) dans chacune des images radiographiques 2D.

3. Procédé selon la revendication 1 ou la revendication 2, dans lequel l'image médicale 3D est reconstruite à partir d'un ensemble d'images 2D acquises par le système d'imagerie à rayons X et les au moins deux images 2D sont sélectionnées dans cet ensemble d'images 2D.

4. Procédé selon la revendication 1 ou la revendication 2, dans lequel l'image médicale 3D est reconstruite à partir d'un ensemble d'images 2D acquises par le système d'imagerie à rayons X et les images 2D sont acquises avec le système d'imagerie à rayons X après la reconstruction de l'image médicale 3D.

5. Procédé selon la revendication 1 ou la revendication 2, dans lequel l'image médicale 3D est reconstruite à partir d'un ensemble d'images 2D acquises par le système d'imagerie à rayons X et les images 2D sont acquises avec le système d'imagerie à rayons X avant la reconstruction de l'image médicale 3D.

6. Méthode selon l'une des revendications 1 à 5, dans laquelle la technique de recalage d'image à image comprend au moins l'une des techniques suivantes : des techniques de corrélation croisée, des techniques d'information mutuelle, des techniques de corrélation de gradient.

7. Système chirurgical intraopératoire comprenant :
- un fantôme de recalage (1) comprenant un ensemble de repères radio-opaques (10) ayant une position connue dans un système de coordonnées dudit fantôme de recalage (1), ledit fantôme de recalage étant adapté pour être positionné sur un patient,
- un système d'imagerie à rayons X configuré pour acquérir un ensemble d'images 2D radiographiques pour reconstruire une image médicale 3D à partir de cet ensemble d'images 2D radiographiques,
- un système informatique configuré pour :
(i) obtenir au moins deux images radiographiques 2D acquises par le système d'imagerie à rayons X, dans lesquelles au moins un repère radio-opaque (10) du fantôme de recalage (1) est détectable sur chaque image 2D ;
(ii) pour chacune de ces au moins deux images 2D, mettre en correspondance l'image radiographique 2D avec le fantôme de recalage (1) en détectant et en déterminant la position d'au moins un repère radio-opaque (10) dans l'image 2D correspondante ;
(iii) mettre en correspondance chacune de ces au moins deux images radiographiques 2D avec l'image 3D à l'aide d'une technique de recalage d'image à image ;
(iv) mettre en correspondance le fantôme de recalage (1) avec l'image médicale 3D en utilisant lesdites au moins deux images radiographiques 2D.

8. Système selon la revendication 7, dans lequel le système d'imagerie à rayons X est un arceau motorisé.

9. Système selon la revendication 7 ou la revendication 8, comprenant en outre une base radiotransparente (2) configurée pour être fixée à l'anatomie du patient, ladite base (2) comprenant un système de fixation pour fixer de manière amovible le fantôme de recalage (1) à la base.

10. Système selon l'une des revendications 7 à 9, comprenant en outre un système de localisation et au moins un traqueur adapté pour être suivi par le système de localisation, la base comprenant un système de fixation pour attacher de manière amovible le traqueur à la base.
